# EUROPÄISCHE PATENTSCHRIFT

(11) **EP 3 387 902 B1**
(45) Veröffentlichungstag und Bekanntmachung des Hinweises auf die Patenterteilung: **21.04.2021**
(21) Anmeldenummer: 18166578.7
(22) Anmeldetag: 10.04.2018
(51) Int. Cl.: A01K 31/22, A01K 31/17, A01K 31/16

(54) **GEFLÜGELAUFENTHALTSVORRICHTUNG, INSBESONDERE ZUR EIABLAGE FÜR ENTEN, UND VERFAHREN**
POULTRY ACCOMMODATION, IN PARTICULAR FOR EGG LAYING FOR DUCKS, AND METHOD
DISPOSITIF D'ABRITEMENT DE VOLAILLE, EN PARTICULIER DESTINÉ AU DÉPÔT D' OEUFS POUR CANARDS ET PROCÉDÉ

(30) Priorität: 10.04.2017 DE 202017102141 U
(43) Veröffentlichungstag der Anmeldung: 17.10.2018
(73) Patentinhaber: Big Dutchman International GmbH, 49377 Vechta (DE)
(72) Erfinder: AUFFARTH, Volker, 49360 Vechta (DE)
(74) Vertreter: Eisenführ Speiser

(56) Entgegenhaltungen:
- EP-A1- 2 829 745
- EP-A1- 2 907 381
- US-A- 3 124 102

## Beschreibung

"Die Erfindung betrifft eine Geflügelaufenthaltsvorrichtung, insbesondere zur Eiablage für Geflügeltiere wie Enten oder Hühner, einen Geflügelstall, insbesondere zur Haltung von Geflügeltieren wie Enten oder Hühnern, und ein Verfahren zur Haltung von Geflügeltieren wie Enten oder Hühnern."

Im Stand der Technik sind Vorrichtungen zur Eiablage für Enten grundsätzlich bekannt. In bekannten Vorrichtungen für Enten sind in der Regel Legenester vorgesehen, in denen eine Ente ein Ei legen kann. Von dem Legenest aus kann das Ei im Anschluss manuell, halbautomatisiert oder automatisiert abtransportiert werden. Ein Nachteil der im Stand der Technik bekannten Vorrichtungen zur Eiablage besteht unter anderem darin, dass die Eier nicht oder lediglich in eingeschränktem Maße aus dem Legenest herausgenommen werden können bzw. nicht oder in eingeschränkter Weise aus dem Legenest automatisiert abtransportiert werden können. Insbesondere können unterschiedliche Faktoren den Eiabtransport verhindern bzw. verschlechtern.

Darüber hinaus ist es ein Nachteil von bekannten Vorrichtungen, dass die Nester nicht die gewünschten Lichtverhältnisse aufweisen und somit das Wohlbefinden der Tiere und infolgedessen die Produktivität der Eiablage eingeschränkt ist. Darüber hinaus ist es ein besonderer Nachteil der im Stand der Technik bekannten Vorrichtungen, dass die Geflügelaufenthaltsvorrichtungen regelmäßig verschmutzen. Dies geschieht unter anderem dadurch, dass regelmäßig neuer Einstreu in den Stall eingebracht wird und dieser insbesondere mit zunehmender Menge in die Geflügelaufenthaltsvorrichtung gelangt. Unter anderem daher ist eine regelmäßige Reinigung der Geflügelaufenthaltsvorrichtung, die in der Regel manuell und personalaufwendig durchgeführt werden muss, notwendig.

Darüber hinaus verändern sich durch den Stalleinstreu regelmäßig die Nesteintrittsverhältnisse für die Enten, sodass sich diese ständig an neue Verhältnisse gewöhnen müssen, was wiederum zu einem verminderten Wohlbefinden der Tiere führen kann. Insbesondere Enten können sich an derartige veränderte Bedingungen nur langsam gewöhnen. Darüber hinaus verschmutzt ein derartiger Stall nach der Reinigung schnell wieder. Ferner ist es ein Nachteil der im Stand der Technik bekannten Vorrichtungen, dass die Enten nachts in den Legenestern verweilen können und diese dadurch beschmutzen. Die Haltung von Enten wird infolgedessen teuer und insbesondere vor dem Hintergrund steigender Anforderungen an das Wohlbefinden der Tiere ungeeignet. Neben den grundsätzlich bestehenden Anforderungen an das Wohlbefinden der Tiere resultiert dieses geringe Wohlbefinden ferner in einer eingeschränkten Produktivität der Tiere bei der Eierproduktion.

Aus EP2829745A1ist eine Stallkonstruktion für die Aufzucht von Geflügel bekannt, die über Legenester und einen Rahmen verfügt. An dem Rahmen sind Stützbeine angeordnet, die eine Höhenjustierung zum Ausgleich von Bodenunebenheiten ermöglicht. Nach Ausgleich der Bodenunebeneinheiten werden die Stützbeine durch eine Klemmung mittels Reibschluss und zusätzlich eine Verschraubung mittels Formschluss gesichert. US3124102A und EP2907381A1 zeigen ebenfalls Stallkonstruktionen mit solchen ausfahrbahren und in einer Position sicherbaren Stützbeinen.

Neben den allgemeinen Anforderungen an die Entenhaltung, wie beispielsweise der Stallsauberkeit, dem Wohlbefinden der Tiere sowie geringer Kosten und hoher Produktivität, ist es daher eine Aufgabe der vorliegenden Erfindung, eine Geflügelaufenthaltsvorrichtung zur Eiablage für Enten, einen Geflügelstall zur Haltung von Enten und ein Verfahren zur Haltung von Enten bereitzustellen, welche gleichbleibende Nestverhältnisse ermöglicht.

Gemäß einem ersten Aspekt der vorliegenden Erfindung wird die eingangs genannte Aufgabe gelöst durch eine Geflügelaufenthaltsvorrichtung nach Anspruch 1, insbesondere zur Eiablage für Enten, mit einer Mehrzahl von nebeneinander angeordneten Legenestern, die oberhalb einer Bodenfläche angeordnet sind, mindestens einem Stützelement, das sich in vertikaler Richtung von einem unteren Standende zu einem oberen Freiende erstreckt und in vertikaler Richtung relativ bewegbar zu der Bodenfläche angeordnet ist, und mit einem Verriegelungselement, welches mit dem Stützelement zusammenwirkt, um eine Bewegung des Stützelements in einer vertikal nach oben gerichteten Standrichtung relativ zu der Bodenfläche zu sperren und eine Bewegung des Stützelements relativ zu der Bodenfläche in einer vertikal nach unten gerichteten Freilaufrichtung freizugeben.

Der Erfindung liegt unter anderem die Erkenntnis zugrunde, dass die Produktivität bei der Enteneierproduktion stärker durch das Wohlbefinden der Tiere beeinflusst wird als bei anderen Eier-legenden Tierarten. Darüber hinaus wurde erkannt, dass das Wohlbefinden der Enten dadurch positiv beeinflusst wird, dass diese gleichbleibende Nestverhältnisse vorfinden. Durch die regelmäßige Einbringung von Einstreu wächst jedoch die Höhe des Einstreus im Stall regelmäßig an, sodass sich die Nesteintrittsverhältnisse in der Regel verändern. Die ständige Beseitigung des alten Einstreus vor der Einbringung des neuen Einstreus ist bereits aus wirtschaftlichen Gründen keine Möglichkeit, da der Personalaufwand zu hoch wäre. Ferner ist diese Möglichkeit aus verhaltenstechnischen Gründen in der Regel nicht möglich, da die Tiere durch die regelmäßige Anwesenheit von Menschen in ihrem Wohlbefinden gestört werden.

Die erfindungsgemäße Geflügelaufenthaltsvorrichtung kann mit geringstem manuellen Aufwand oder auch automatisiert an unterschiedliche Einstreuhöhen im Stall angepasst werden, indem das mindestens eine Stützelement bei Anhebung der Geflügelaufenthaltsvorrichtung, beispielsweise durch sein Eigengewicht, ausfährt. Somit kann die Höhe der Bodenfläche und der Geflügelaufenthaltsvorrichtung oberhalb einer Aufstellfläche, auf der die Stützelemente mit dem Standende aufstehen, verändert werden und daher der Abstand der Bodenfläche zu einem auf der Aufstellfläche anwachsenden Einstreuhaufen konstant gehalten werden. Infolgedessen ist auch der Verschmutzungsgrad innerhalb der Geflügelaufenthaltsvorrichtung kontrollierbar und das Wohlbefinden der Tiere kann weiter gesteigert werden.

Im Betriebsmodus ist die Bodenfläche vorzugsweise im Wesentlichen horizontal ausgerichtet. Die Bodenfläche kann als eine durchgängige Fläche oder durch zwei oder mehrere einzelne Bodenflächenelemente ausgebildet werden. Die Bodenfläche erstreckt sich in Längsrichtung und Breitenrichtung vorzugsweise von den jeweils gegenüberliegenden Endseiten der Geflügelaufenthaltsvorrichtung.

In einer bevorzugten Fortbildung ist vorgesehen, dass die Geflügelaufenthaltsvorrichtung ein Rahmengestell aufweist. Das Rahmengestell weist vorzugsweise zwei parallele Rahmenelemente in Längsrichtung der Geflügelaufenthaltsvorrichtung und zwei parallele Rahmenelemente in Breitenrichtung auf. Die Bodenfläche kann beispielsweise an dem Rahmengestell befestigt sein. Das Rahmengestell kann Quer- und/oder Längsstreben aufweisen, die die Stabilität der Geflügelaufenthaltsvorrichtung verbessern und an denen die Legenester angeordnet sein können. Die Rahmenelemente des Rahmengestells bestehen vorzugsweise aus Stahl, und/oder Aluminium, und/oder Kunststoff..

Die einzelnen Legenester umfassen vorzugsweise jeweils eine erste Seitenwand und eine zweite Seitenwand. Die erste Seitenwand und die zweite Seitenwand sind vorzugsweise flächenparallel ausgerichtet. Ferner sind die erste Seitenwand und die zweite Seitenwand vorzugsweise senkrecht zu der Bodenfläche angeordnet. Die erste und die zweite Seitenwand erstrecken sich vorzugsweise parallel zur Breitenrichtung der Geflügelaufenthaltsvorrichtung. Insbesondere ist es bevorzugt, dass die Legenester jeweils durch Seitenwände seitlich begrenzt werden und dass zwischen einem ersten Legenest und einem zweiten Legenest die Seitenwand durch eine Trennwand gebildet wird. Darüber hinaus ist es bevorzugt, dass die Legenester entlang der Längsrichtung der Geflügelaufenthaltsvorrichtung nebeneinander angeordnet sind. Darüber hinaus ist es bevorzugt, dass die Geflügelaufenthaltsvorrichtung ein Dachelement aufweist, das die Legenester teilweise oder im Wesentlichen vollständig überdeckt.

Die in Längsrichtung nebeneinander angeordneten Legenester können somit auch eine Legenestreihe bilden. Darüber hinaus kann es bevorzugt sein, dass die Geflügelaufenthaltsvorrichtung eine erste Legenestreihe und eine zweite Legenestreihe aufweist. Insbesondere ist es bevorzugt, dass die erste Legenestreihe und die zweite Legenestreihe in Breitenrichtung voneinander beabstandet sind, und/oder zwischen der ersten Legenestreihe und der zweiten Legenestreihe eine Eiertransportvorrichtung angeordnet ist. Darüber hinaus kann es bevorzugt sein, dass die erste Legenestreihe die gleiche Anzahl an Legenestern aufweist wie die zweite Legenestreihe, und/oder die erste Legenestreihe und die zweite Legenestreihe in Längsrichtung die gleiche Abmessung aufweisen. Ferner kann es bevorzugt sein, dass zwischen der ersten Legenestreihe und der zweiten Legenestreihe im Wesentlichen kein Abstand vorhanden ist. Darüber hinaus kann es bevorzugt sein, dass die Bodenfläche eine oder mehrere Durchtrittsöffnungen aufweist, sodass gelegte Eier von der Bodenfläche in einen Bereich unterhalb der Legenester gelangen und von hier aus abtransportiert werden können. Eine hierzu unterhalb oder fluchtend zu der Bodenfläche vorgesehene Eiertransportvorrichtung kann manuell oder motorisch, beispielsweise mittels eines elektrischen Antriebs, angetrieben sein.

Vorzugsweise weist das mindestens eine Stützelement ein derartiges Eigengewicht auf, dass das Stützelement bei Nichtbelastung durch die Geflügelaufenthaltsvorrichtung, also wenn das Standende unbelastet ist, durch das Eigengewicht ausfährt, insbesondere unter seinem Eigengewicht auch gegen einen mechanischen Widerstand der Sperreinrichtung ausfährt. Ferner ist es bevorzugt, dass das untere Standende des Stützelementes an der Aufstellfläche befestigt ist.

Das mindestens eine Stützelement erstreckt sich in vertikaler Richtung von dem unteren Standende zu dem oberen Freiende. Als Erstreckungsrichtung des Stützelements in vertikaler Richtung wird auch verstanden, dass eine Richtungskomponente der Erstreckungsrichtung in vertikaler Richtung verläuft. Das Stützelement kann also beispielsweise auch schräg angeordnet werden, so dass die Erstreckungsrichtung zwischen dem unteren Standende und dem oberen Freiende eine vertikale Richtungskomponente und eine horizontale Richtungskomponente aufweist.

Vorzugsweise weist das Stützelement einen Querschnitt auf, der dreieckig, viereckig und/oder polygonal ausgebildet ist. Darüber hinaus kann der Querschnitt auch rund, mit einem runden Umfangsabschnitt und/oder elliptisch ausgebildet sein. Darüber hinaus ist es bevorzugt, dass der Querschnitt eine langgezogene rechteckige Ausbildung aufweist, sodass das Stützelement plattenförmig ausgebildet ist. Vorzugsweise ist das Stützelement an der Geflügelaufenthaltsvorrichtung derart angeordnet, dass dessen Erstreckungsrichtung oder eine Richtungskomponente der Erstreckungsrichtung im Wesentlichen parallel zu einer Flächennormalen der Bodenfläche ausgerichtet ist.

Ferner ist es bevorzugt, dass die Geflügelaufenthaltsvorrichtung zwei oder mehrere Stützelemente aufweist. Das mindestens eine Stützelement ist ferner bewegbar angeordnet. Beispielsweise kann die Geflügelaufenthaltsvorrichtung eine Führungseinrichtung aufweisen, in der das Stützelement entlang seiner Erstreckungsrichtung geführt ist. Insbesondere ist es bevorzugt, dass die Führungsvorrichtung rohrförmig ausgebildet ist.

Durch die bewegbare Anordnung des mindestens einen Stützelements weist dieses mindestens eine Bewegungsrichtung auf. Zumindest eine Richtungskomponente dieser Bewegungsrichtung ist im Betriebsmodus vertikal ausgerichtet. Das heißt insbesondere, dass das Stützelement von der Geflügelaufenthaltsvorrichtung in Richtung der Aufstellfläche ausfahrbar ist. Infolgedessen kann das untere Standende des Stützelements von der Bodenfläche weg bewegt werden, sodass der Abstand zwischen dem unteren Standende und den Legenestern und/oder der Bodenfläche veränderbar, insbesondere vergrößerbar, ist. Infolgedessen kann der Abstand zwischen den oberhalb der Bodenfläche angeordneten Legenestern und der Aufstellfläche vergrößert werden. Vorzugsweise ist die Bewegungsrichtung parallel zu einer Flächennormalen der Bodenfläche ausgerichtet.

Das untere Standende des Stützelements ist im Betriebsmodus der Aufstellfläche, wie einem Stallboden, zugewandt, auf der die Geflügelaufenthaltsvorrichtung aufgestellt ist. Das obere Freiende ist in Bezug auf das untere Standende der Aufstellfläche abgewandt. Das mindestens eine Stützelement ist in seiner Erstreckungsrichtung ferner derart angeordnet und ausgebildet, dass dieses in der Standrichtung von dem unteren Standende zu dem oberen Freiende im Wesentlichen durch eine vorzugsweise lösbare Arretierung unbewegbar ist. Das bedeutet, sobald eine Kraft am unteren Standende in Richtung des oberen Freiendes angreift, dass das Stützelement durch diese Kraft im Wesentlichen nicht bewegt wird. Das Stützelement ist beispielsweise derart eingerastet, dass dieses in der Standrichtung nicht bewegbar ist.

Darüber hinaus ist das Stützelement derart angeordnet und ausgebildet, dass dieses in der Freilaufrichtung bewegbar ist. Wenn demnach eine Kraft an dem Stützelement von dem oberen Freiende in Richtung des unteren Standendes angreift, wird das Stützelement in seiner Erstreckungsrichtung bewegt. Dadurch kann das Stützelement durch das Angreifen einer Kraft in Freilaufrichtung ausgefahren werden. Vorzugsweise ist das Stützelement in seiner Erstreckungsrichtung derart angeordnet und ausgebildet, dass die Gewichtskraft des Stützelements ausreicht, um eine Bewegung in Freilaufrichtung zu ermöglichen, sobald keine Kraft in Standrichtung wirkt bzw. eine Kraft in Freilaufrichtung, beispielsweise durch das Eigengewicht, größer ist als eine Kraft in Standrichtung, beispielsweise durch eine Reibkraft. Eine Realisierung eines im Vorherigen beschriebenen Stützelements erfolgt durch einen Freilauf, insbesondere einen Linearfreilauf. Das Stützelement besteht vorzugsweise aus Stahl oder umfasst diesen. Ferner vorzugsweise besteht das Stützelement aus Aluminium oder Kunststoff oder umfasst diesen bzw. diese.

Der Freilauf sperrt also ein Einfahren des Stützelements in Standrichtung und ermöglicht ein Ausfahren des Stützelements in Freilaufrichtung. Hierdurch kann die Höhe der Bodenfläche und der Legenester oberhalb einer Aufstellfläche, auf der die Geflügelaufenthaltsvorrichtung aufgestellt ist, einem wachsenden Einstreuhaufen unterhalb und/oder neben der Geflügelaufenthaltsvorrichtung angepasst werden. Die Sperrwirkung des Freilaufs gegen Einfahren ist vorzugsweise lösbar, um nach Entfernen des gewachsenen Einstreuhaufens die Geflügelaufenthaltsvorrichtung wieder absenken zu können. Die Sperrwirkung kann aber auch unlösbar sein, in diesem Falle können die Stützelemente so ausgebildet sein, dass sie nach unten aus ihrer Führungsvorrichtung herausgezogen werden können und dann von oben wieder in ihre Führungsvorrichtung eingesetzt werden zu können, um nach einem vollständig ausgefahrenen Zustand einen vollständig eingefahrenen Zustand zu erreichen.

Vorzugsweise ist die Geflügelaufenthaltsvorrichtung gekennzeichnet durch eine sich entlang einer Seite im Bereich der Bodenfläche erstreckende Seitenschiene, die eine oder mehrere Aussparungen im Bereich der Legenester aufweist. Seitenschienen werden insbesondere in Geflügelaufenthaltsvorrichtungen für Hühnervögel vorgesehen, wobei ein Hühnervogel zum Eintreten in die Geflügelaufenthaltsvorrichtung zunächst auf die Seitenschiene und abschließend hinab in das Legenest zu steigen hat. Eine durch eine ausgesparte Seitenschiene gekennzeichnete Geflügelaufenthaltsvorrichtung weist insbesondere den Vorteil auf, dass der Nesteintritt für Enten vereinfacht ist. Dies liegt insbesondere in der anatomischen Ausführung der Entenfüße begründet, da diese besonderes breit ausgebildet sind.

Durch die erfindungsgemäße Geflügelaufenthaltsvorrichtung können konstante Nesteintrittsverhältnisse auch bei kontinuierlichem Einbringen von Einstreu in einen Stall ermöglicht werden. Beispielsweise kann die Geflügelaufenthaltsvorrichtung an einer Nesthebevorrichtung angeordnet werden, die die Geflügelaufenthaltsvorrichtung in vertikaler Richtung anhebt. Durch dieses Anheben wird ein Ausfahren der Stützelemente ermöglicht, beispielsweise kann bei entsprechender Ausbildung der Anordnung des Stützelements und entsprechendem Gewicht des Stützelements das Stützelement automatisch ausgefahren. Dies wird durch die bewegbare Anordnung des Stützelements in Freilaufrichtung ermöglicht. Nachdem die Geflügelaufenthaltsvorrichtung in die gewünschte Position bzw. Höhe über der Aufstellfläche gebracht wurde, kann beispielsweise die Nesthebevorrichtung wieder kraftlos geschaltet werden und die Geflügelaufenthaltsvorrichtung stützt sich auf dem mindestens einen Stützelement ab, da dieses in Standrichtung im Wesentlichen unbewegbar angeordnet ist. Somit kann innerhalb von kürzester Zeit die Höhe der Geflügelaufenthaltsvorrichtung über der Aufstellfläche angepasst und ein stabiler Stand erreicht werden. Darüber hinaus kann eine Vielzahl von Geflügelaufenthaltsvorrichtungen innerhalb kürzester Zeit in ihrer Höhe verstellt werden. Wie im Folgenden noch näher erläutert wird, ist in einer bevorzugten Ausführungsvariante auch eine Absenkung der Geflügelaufenthaltsvorrichtung durch ein Entsperrelement möglich.

Es ist darüber hinaus vorteilhaft, dass zur Einstellung der Höhe kein Bediener den Stall betreten muss. Dies spart Personalkosten und Personalaufwand, aber resultiert ferner in einer Verbesserung des Wohlbefindens der Tiere. Durch den Eintritt eines Bedieners in den Stall werden die Tiere, insbesondere Enten, gestört, was in Stress resultieren kann. Darüber hinaus müssen die Geflügelaufenthaltsvorrichtungen seltener gereinigt werden, sodass auch hier eine Reduktion von Personalkosten und -aufwänden resultiert und das Wohlbefinden der Tiere noch weiter gesteigert wird.

Die hochziehbare Geflügelaufenthaltsvorrichtung hat den weiteren Vorteil, dass die eine oder auch mehrere Geflügelaufenthaltsvorrichtungen mit geringem Aufwand so weit hochgezogen werden können, dass die Stützelemente maximal ausgefahren sind oder sogar von der Aufstellfläche abgehoben werden und dieser angehobenen Position der Geflügelaufenthaltsvorrichtungen der Stall ohne großen Aufwand gereinigt werden kann.

In einer bevorzugten Ausführungsvariante der Geflügelaufenthaltsvorrichtung ist vorgesehen, dass die Bodenfläche durch eine Gitter- und/oder Streben- und/oder Gestellanordnung ausgebildet ist und eine drei- oder mehreckige Geometrie aufweist, wobei vorzugsweise in einem Bereich angrenzend an eine erste Ecke ein erstes Stützelement, und/oder an eine zweite Ecke ein zweites Stützelement, und/oder an eine dritte Ecke ein drittes Stützelement, und/oder an eine vierte Ecke ein viertes Stützelement angeordnet ist.

Die Stützelemente können vorzugsweise auch vollständig oder teilweise direkt an den Ecken angeordnet sein. Darüber hinaus können die Stützelemente auch im Bereich von Kanten angeordnet sein. Insbesondere ist es bevorzugt, dass die Stützelemente an Kanten angeordnet sind, die sich zwischen der ersten, und/oder zweiten, und/oder dritten und/oder vierten Ecke erstrecken. Ferner besteht die Möglichkeit, dass die Stützelemente unter der Bodenfläche angeordnet sind. Ferner ist es bevorzugt, dass das mindestens eine Stützelement parallel zu einer Flächennormalen der Bodenfläche bewegbar ist. Ferner vorzugsweise ist die Erstreckungsrichtung des mindestens einen Stützelements parallel zu einer Flächennormalen der Bodenfläche des Gestells ausgerichtet.

Gemäß einer weiteren bevorzugten Ausführungsvariante der Geflügelaufenthaltsvorrichtung ist vorgesehen, dass das mindestens eine Stützelement mittels einer Sperreinrichtung gegen eine Bewegung entlang seiner Erstreckungsrichtung gehalten ist, wobei die Sperreinrichtung vorzugsweise mindestens eine federnd gelagerte Klinke, insbesondere einen Linearfreilauf, und/oder mindestens ein bewegbares Schnappelement, und/oder mindestens einen Reibwiderstand, und/oder mindestens eine Hydraulikvorrichtung, und/oder eine Bremsvorrichtung, vorzugsweise umfassend Bremsbacken, umfasst.

Bei mindestens einer federnd gelagerten Klinke, weist das Stützelement vorzugsweise korrespondierend zur Klinke ausgebildete Ausnehmungen auf. Die Klinke und die Ausnehmungen sind vorzugsweise derart ausgebildet, dass die Unbeweglichkeit in Standrichtung und die Beweglichkeit in Freilaufrichtung des Stützelements gewährleistet ist. Auch bei dem mindestens einen bewegbaren Schnappelement ist es bevorzugt, dass das Stützelement korrespondierend ausgebildete Ausnehmungen aufweist, so dass das Stützelement in Standrichtung im Wesentlichen unbeweglich ist und in Freilaufrichtung bewegbar. Alternativ zu den zuvor genannten Ausnehmungen können auch Verdickungen, Wölbungen oder anordenbare Elemente an dem Stützelement angeordnet werden. Alternativ kann auch ein Reibwiderstand vorgesehen werden, um die Unbeweglichkeit des Stützelements in Standrichtung und die Beweglichkeit des Stützelements in Freilaufrichtung zu ermöglichen. Beispielsweise kann die Sperreinrichtung mit dem Stützelement eine Reibflächenpaarung bilden, wobei ein Reibkoeffizient in Bewegungsrichtung des Stützelements anisotrop ausgebildet ist. Somit kann beispielsweise ein Reibwiderstand zwischen der Sperreinrichtung und dem Stützelement in Standrichtung hoch und in Freilaufrichtung niedrig ausgebildet werden. Infolgedessen kann die Beweglichkeit in Freilaufrichtung und die Unbeweglichkeit in Standrichtung realisiert werden. Durch die Bremsbacken wird vorzugsweise aufgrund einer winkligen Zwangsführung eine Klemmung des Stützelementes ermöglicht, wobei diese Klemmung lediglich in Standrichtung erfolgt und nicht in Freilaufrichtung. Bei dem Einsatz der Hydraulikvorrichtung ist es insbesondere bevorzugt, dass die Verstellung des Stützelementes stufenlos erfolgt. Bei den im Vorherigen beschriebenen Ausführungsvarianten für die Sperreinrichtung ist es besonders bevorzugt, dass diese jeweils eine Entsperreinrichtung umfasst, mittels derer eine Bewegung des Stützelements in Standrichtung ermöglicht wird.

Diese Ausführungsvariante der Geflügelaufenthaltsvorrichtung hat den besonderen Vorteil, dass das Stützelement in sicherer Weise bewegbar in Freilaufrichtung und im Wesentlichen unbewegbar in Standrichtung angeordnet werden kann. Insbesondere besteht hiermit die Möglichkeit, eine sichere und robuste Realisierung der Geflügelaufenthaltsvorrichtung zu gewährleisten. Ferner können geringe Produktionskosten realisiert werden.

Eine weitere bevorzugte Fortbildung der Geflügelaufenthaltsvorrichtung zeichnet sich dadurch aus, dass diese eine Führungsvorrichtung für das mindestens eine Stützelement umfasst, wobei die Führungsvorrichtung insbesondere eine rohrförmige Geometrie mit einem Innenquerschnitt aufweist, das mindestens eine Stützelement einen Außenquerschnitt aufweist, wobei der Innenquerschnitt der Führungsvorrichtung und der Außenquerschnitt des Stützelements insbesondere die gleiche Geometrie aufweisen und der Innenquerschnitt der Führungsvorrichtung und der Außenquerschnitt des Stützelements insbesondere derart ausgebildet sind, dass das mindestens eine Stützelement bewegbar innerhalb der Führungsvorrichtung anordenbar ist. Eine derartige Führung des Stützelements mittels einer Führungsvorrichtung ermöglicht das sichere Bewegen der Geflügelaufenthaltsvorrichtung. Insbesondere besteht mit einer derartigen Führung die Möglichkeit, dass die Geflügelaufenthaltsvorrichtung mittels einer labilen Nesthebevorrichtung angehoben werden kann und dennoch im Anschluss ein sicherer Stand gewährleistet werden kann, da die Nesthebevorrichtung entlastet werden kann und keine dauerhaften Kräfte aufnehmen muss, die stattdessen durch das mindestens eine geführte Stützelement aufgenommen werden. Somit besteht die Möglichkeit einer einfachen und kostengünstigen Anhebung der Geflügelaufenthaltsvorrichtung. Vorzugsweise ist für jedes Stützelement eine Führungsvorrichtung angeordnet.

Ferner ist vorzugsweise vorgesehen, dass das mindestens eine Stützelement an mindestens einer Außenseite Aussparungen aufweist, die mindestens eine federnd gelagerte Klinke ein von einem Drehpunkt beabstandetes Einrastende aufweist, wobei das Einrastende in den Aussparungen anordenbar ist, und vorzugsweise durch eine Feder in Richtung der Aussparungen mit einer Kraft beaufschlagt ist, und die Aussparungen und die Klinke derart angeordnet und ausgebildet sind, dass das mindestens eine Stützelement in Standrichtung im Wesentlichen nicht bewegbar ist, wobei das Einrastende bei Bewegung des mindestens einen Stützelements in Standrichtung in einer Aussparung einrastet, und das mindestens eine Stützelement in Freilaufrichtung bewegbar ist, wobei das Einrastende bei Bewegung des mindestens einen Stützelements in Freilaufrichtung nicht in einer Aussparung einrastet.

Eine Kombination einer Klinke mit korrespondierend ausgebildeten Ausnehmungen resultiert in dem Vorteil einer einfachen und robusten Sperreinrichtung, die einfach zu warten ist und darüber hinaus eine geringe Ausfallwahrscheinlichkeit bietet. Darüber hinaus sind die Herstellkosten einer derartigen Geflügelaufenthaltsvorrichtung gering. Insbesondere ist es bevorzugt, dass die Feder eine derartige Federkraft aufweist, dass das Stützelement durch sein Eigengewicht in Freilaufrichtung bei Nichtbelastung durch die Geflügelaufenthaltsvorrichtung die Klinke aus einer eingerasteten Position wegbewegen kann und dann in Freilaufrichtung ausfahren kann.

In einer weiteren bevorzugten Fortbildung der Geflügelaufenthaltsvorrichtung umfasst diese ein Entsperrelement, das angeordnet und ausgebildet ist, eine Bewegung des mindestens einen Stützelements in Standrichtung zu ermöglichen. Dabei ist es insbesondere bevorzugt, dass das Entsperrelement an der federnd gelagert Klinke angreift und angeordnet und ausgebildet ist, das Einrastende von dem Stützelement zu beabstanden.

Eine weitere bevorzugte Ausführungsvariante der Geflügelaufenthaltsvorrichtung sieht vor, dass in einem Standmodus das untere Standende des mindestens einen Stützelements auf einer Aufstellfläche angeordnet ist, das Gewicht der Geflügelaufenthaltsvorrichtung zumindest teilweise auf dem mindestens einen Stützelement lastet und das mindestens eine Stützelement die Geflügelaufenthaltsvorrichtung mit einem definierten Abstand über der Aufstellfläche abstützt, und in einem Einstellmodus das Gewicht der Geflügelaufenthaltsvorrichtung im Wesentlichen nicht auf dem mindestens einen Stützelement lastet und das mindestens eine Stützelement in Freilaufrichtung im Wesentlichen durch Eigengewicht bewegbar angeordnet ist. In dem Einstellmodus kann das Gewicht der Geflügelaufenthaltsvorrichtung durch eine Haltevorrichtung gehalten werden, die beispielsweise mittels eines Seils an der Geflügelaufenthaltsvorrichtung realisiert ist. Dadurch wird das oder die Stützelemente entlastet, und bei Anheben der Geflügelaufenthaltsvorrichtung wird das Stützelement, beispielsweise durch sein Eigengewicht, ausgefahren, sodass das untere Standende im Wesentlichen auf einer Aufstellfläche verbleibt bzw. sich durch Eigengewicht wieder zu der Aufstellfläche hin bewegt. Sobald die Gewichtskraft der Geflügelaufenthaltsvorrichtung wieder auf dem Stützelement lastet, stützt sich die Geflügelaufenthaltsvorrichtung mit einem durch den Ausfahrzustand des(der Stützenelemente definierten Abstand über der Aufstellfläche alleinig über das/die Stützenelemente ab.

In einer weiteren bevorzugten Ausführungsvariante der Geflügelaufenthaltsvorrichtung ist vorgesehen, dass diese ein Entsperrelement umfasst, das angeordnet und ausgebildet ist, eine Bewegung des mindestens einen Stützelements in Standrichtung zu ermöglichen. Durch das Entsperrelement wird die Bewegung des mindestens einen Stützelements in Standrichtung ermöglicht, wodurch der Abstand der Geflügelaufenthaltsvorrichtung zu einer Aufstellfläche reduziert werden kann. Nachdem beispielsweise ein Stall von Einstreu befreit wurde, können somit die Geflügelaufenthaltsvorrichtungen einzeln oder bei der Anordnung mehrerer Geflügelaufenthaltsvorrichtungen auch gemeinsam wieder abgesenkt werden. Das Entsperrelement kann hierbei insbesondere mit einer Sperreinrichtung der zuvor beschriebenen Art zusammenwirken und die Sperrwirkung durch Aufheben einer Bremsoder Rastwirkung aufheben. Bei dem Einsatz mindestens einer federnd gelagerten Klinke oder anderen, durch eine Zustellbewegung auf die das Stützelement wirkenden Sperreinrichtungen kann das Entsperrelement beispielsweise als Seilzugsystem ausgebildet sein. Das Entsperrelement kann ausgebildet sein, um das Einrasten der Klinke zu verhindern oder um eine eingerastete Klinke aus der Einrastung zu lösen. Das Entsperrelement kann mittels einer Handwinde oder motorisch betrieben sein. Insbesondere können mehrere Sperreinrichtungen, beispielsweise mehrere Klinken, durch eine einzige, zentrale Handwinde oder einen einzigen zentralen Motor entsperrt werden. Insbesondere ist es bevorzugt, dass die Sperreinrichtung mindestens eine federnd gelagerte Klinke umfasst, und das Entsperrelement angeordnet und ausgebildet ist, ein Ausrasten der Klinke aus der Aussparung zu bewirken, wobei das Ausrasten vorzugsweise zentral, beispielsweise über einen Zugmechanismus, auslösbar ist.

Gemäß einer weiteren bevorzugten Ausführungsvariante der Geflügelaufenthaltsvorrichtung ist vorgesehen, dass diese ein Brückenelement umfasst, das angeordnet und ausgebildet ist, einem Geflügeltier, insbesondere einer Ente, den Zugang zu der Bodenfläche zu ermöglichen, wobei das Brückenelement vorzugsweise eine flächige und/oder gestufte Oberfläche aufweist und sich ferner vorzugsweise von einer Kante der Geflügelaufenthaltsvorrichtung ausgehend nach schräg unten erstreckt. Vorzugsweise erstreckt sich das Brückenelement parallel zur Längsrichtung der Geflügelaufenthaltsvorrichtung. Vorzugsweise ist das Brückenelement als Lochblech ausgebildet. Darüber hinaus ist es bevorzugt, dass das Brückenelement aus einem Kunststoff besteht oder diesen umfasst und/oder eine maschenförmige und/oder gelochte Oberfläche aufweist. Ferner ist es bevorzugt, dass die Stege der maschenförmigen Oberfläche abgerundet sind, sodass sich die Tiere, insbesondere die Enten, nicht hieran schneiden können. Ferner vorzugsweise ist das Brückenelement demontierbar und/oder weist eine Einhängvorrichtung auf. Das Brückenelement kann insbesondere um eine horizontale Achse schwenkbar an der Geflügelaufenthaltsvorrichtung befestigt sein, um hierdurch einer Hebe- und Senkbewegung der Geflügelaufenthaltsvorrichtung folgen zu können, ohne dass ein auf der Aufstellfläche aufliegendes Ende des Brückenelements von der Aufstellfläche abhebt.

Eine weitere bevorzugte Fortbildung der Geflügelaufenthaltsvorrichtung zeichnet sich dadurch aus, dass diese ein Überdeckungselement umfasst, wobei das Überdeckungselement im Betriebsmodus zumindest abschnittsweise in vertikaler Richtung über der Bodenfläche angeordnet ist und vorzugsweise zwischen dem Überdeckungselement und der Bodenfläche eine lichte Höhe durch eine bewegbare Anordnung des Überdeckungselements veränderbar ist, wobei die lichte Höhe insbesondere in einem Nesteingangsbereich veränderbar ist. Vorzugsweise umfasst das Überdeckungselement bewegbare und nicht bewegbare Abschnitte. Insbesondere ist es bevorzugt, dass bewegbare Überdeckungselemente über den Nesteingangsbereichen angeordnet sind.

Es ist ferner bevorzugt, dass an einer Seite des mindestens einen Legenests eine Eiersammelvorrichtung angeordnet ist, wobei Legeabschnitte der Legenester in vertikaler Richtung von der Eiersammelvorrichtung beabstandet sind, wobei ein Gefälle von dem Legeabschnitt zu der Eiersammelvorrichtung einstellbar ausgebildet ist. Insbesondere ist es bevorzugt, dass die Legenester jeweils zumindest abschnittsweise als ein um mindestens eine horizontale Achse kippbares Element ausgebildet sind. Insbesondere ist es bevorzugt, dass der Legeabschnitt auf bzw. an dem kippbaren Element angeordnet ist bzw. dieses ausbildet. Durch ein kippbares Element als Legeabschnitt kann der Legeabschnitt mit einem Winkel zu einer horizontalen Fläche angeordnet werden. Durch ein derartiges Gefälle kann ein gelegtes Ei schwerkraftbedingt aus dem Legenest in Richtung der Eiersammelvorrichtung abrollen. Insbesondere ist es bevorzugt, dass dieses Gefälle mit einem Winkel von kleiner 7°, 8°, 9° oder 10° und größer als 5°, 6° 7° oder 8°ausgebildet ist. Ein derartig einstellbares kippbares Element hat den besonderen Vorteil, dass die Geflügelaufenthaltsvorrichtung an die individuellen Bedürfnisse der Tiere angepasst werden kann. Insbesondere besteht somit eine Möglichkeit an die Anpassung der Größe der Tiere und/oder der gelegten Eier.

In einer weiteren bevorzugten Fortbildung der Geflügelaufenthaltsvorrichtung ist vorgesehen, dass diese eine Nestzugangsbarriere umfasst, die angeordnet und ausgebildet ist, um den Zugang zu mindestens einem der Legenester für die Geflügeltiere zu versperren. Vorzugsweise ist die Nestzugangsbarriere mit einer Betätigungsvorrichtung mechanisch gekoppelt, die eine Betätigung der Nestzugangsbarriere von einem von der Geflügelaufenthaltsvorrichtung beabstandeten Ort ermöglicht. Insbesondere ist es bevorzugt, dass die Geflügelaufenthaltsvorrichtung ein Barrierezugelement aufweist, das angeordnet und ausgebildet ist, die Nestzugangsbarriere zu bewegen. Insbesondere ist es bevorzugt, dass das Barrierezugelement ein Seil und/oder ein Draht ist, dass durch das Dach hindurchgeführt ist.
Gemäß einem weiteren Aspekt der Erfindung wird die eingangs genannte Aufgabe gelöst durch einen Geflügelstall nach Anspruch 12, insbesondere zur Produktion von Enteneiern, umfassend eine Geflügelaufenthaltsvorrichtung nach mindestens einer der im Vorhergehenden beschriebenen Ausführungsvarianten, wobei die Geflügelaufenthaltsvorrichtung an einer Nesthebevorrichtung angeordnet ist, die angeordnet und ausgebildet ist, die Geflügelaufenthaltsvorrichtung relativ zu einer Aufstellfläche derart zu bewegen, dass ein Abstand zu dieser Aufstellfläche vergrößert wird. Durch einen derartigen Geflügelstall wird das Wohlbefinden der Tiere in besonderer Weise gesteigert und der Personalaufwand und die Personalkosten reduziert.
Gemäß einem weiteren Aspekt der vorliegenden Erfindung wird die eingangs genannte Aufgabe gelöst durch ein Verfahren nach Anspruch 13 zur Produktion von Enteneiern, umfassend Bereitstellen einer Geflügelaufenthaltsvorrichtung nach mindestens einer der im Vorhergehenden beschriebenen Ausführungsvarianten und/oder eines Geflügelstalls nach mindestens einer der im Vorhergehenden beschriebenen Ausführungsvarianten, Bewegen der Geflügelaufenthaltsvorrichtung in einem Einstellmodus in einer im Wesentlichen vertikalen Richtung, wobei ein Abstand zwischen einer Aufstellfläche und der Bodenfläche vergrößert wird, wobei das mindestens eine Stützelement durch sein Eigengewicht im Einstellmodus ausfährt und die Geflügelaufenthaltsvorrichtung in einem anschließenden Standmodus mit einem definierten Abstand zwischen der Aufstellfläche und der Bodenfläche abstützt.
Das erfindungsgemäße Verfahren und seine möglichen Fortbildungen weisen Merkmale bzw. Verfahrensschritte auf, die sie insbesondere dafür geeignet machen, für eine erfindungsgemäße Geflügelaufenthaltsvorrichtung und ihre Fortbildungen verwendet zu werden. Für weitere Vorteile, Ausführungsvarianten und Ausführungsdetails dieser weiteren Aspekte und ihrer möglichen Fortbildungen wird auch auf die zuvor erfolgte Beschreibung zu den entsprechenden Merkmalen und Fortbildungen der Geflügelaufenthaltsvorrichtung verwiesen.

Bevorzugte Ausführungsformen der Erfindung werden beispielhaft anhand der beiliegenden Figuren erläutert. Es zeigen:
- Fig. 1:: eine schematische, dreidimensionale Ansicht einer beispielhaften Ausführungsform einer Geflügelaufenthaltsvorrichtung;
- Fig. 2:: eine schematische, zweidimensionale Vorderansicht der Geflügelaufenthaltsvorrichtung aus Fig. 1;
- Fig. 3:: eine weitere schematische, zweidimensionale Seitenansicht der Geflügelaufenthaltsvorrichtung aus Fig. 1;
- Fig. 4:: eine schematische, dreidimensionale Ansicht einer Sperreinrichtung der Geflügelaufenthaltsvorrichtung aus Fig. 1;
- Fig. 5:: eine schematische, dreidimensionale Ansicht einer beispielhaften Ausführungsform einer Verstellvorrichtung einer Nestzugangsbarriere der Geflügelaufenthaltsvorrichtung aus Fig. 1;
- Fig. 6:: eine weitere schematische, dreidimensionale Ansicht der Verstellvorrichtung für die Nestzugangsbarriere der Geflügelaufenthaltsvorrichtung aus Fig. 1;
- Fig. 7:: eine schematische, dreidimensionale Ansicht einer beispielhaften Ausführungsform eines kippbaren Legeelements der Geflügelaufenthaltsvorrichtung aus Fig. 1;
- Fig. 8:: eine schematische, dreidimensionale Detailansicht einer beispielhaften Ausführungsform einer Geflügelaufenthaltsvorrichtung;
- Fig. 9:: eine weitere schematische, zweidimensionale Vorderansicht der Geflügelaufenthaltsvorrichtung aus Fig. 1;
- Fig. 10:: eine schematische, zweidimensionale Detailansicht eines Kantenschutzelements;

- Fig. 11:: eine schematische, dreidimensionale Ansicht der Geflügelaufenthaltsvorrichtung aus Fig. 1, wobei die Stützelemente im ausgefahrenen Zustand gezeigt sind;
- Fig. 12:: eine schematische, dreidimensionale Detailansicht eines Entsperrmechanismus.

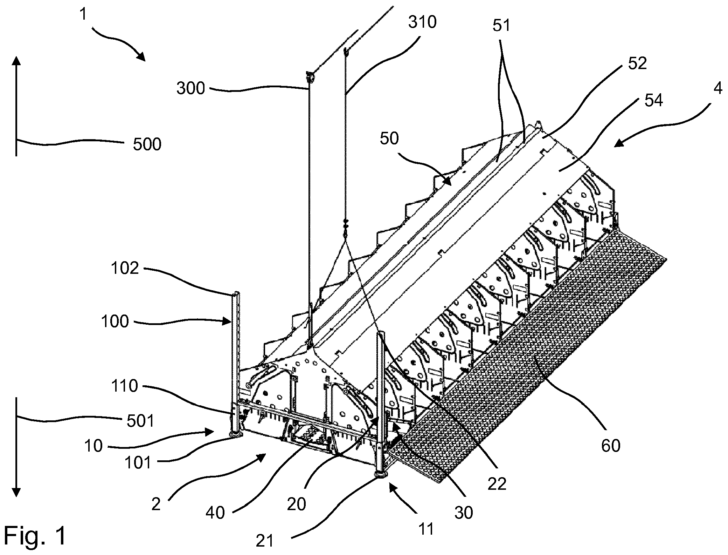

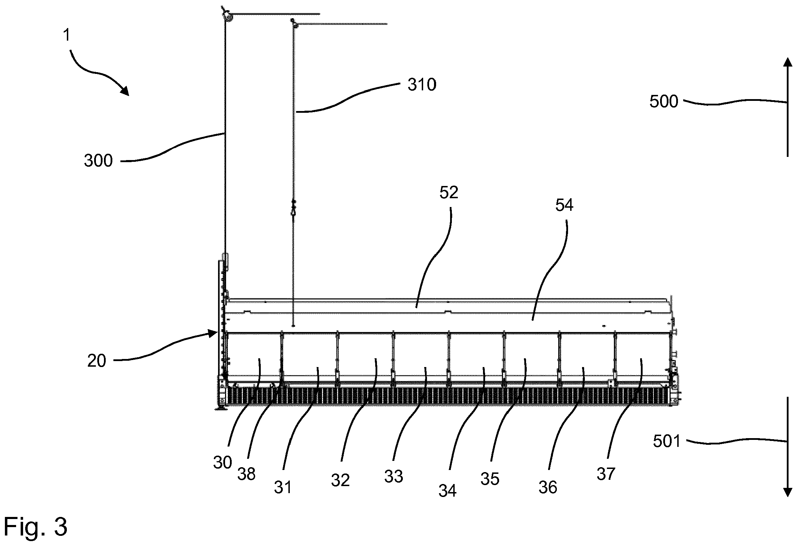

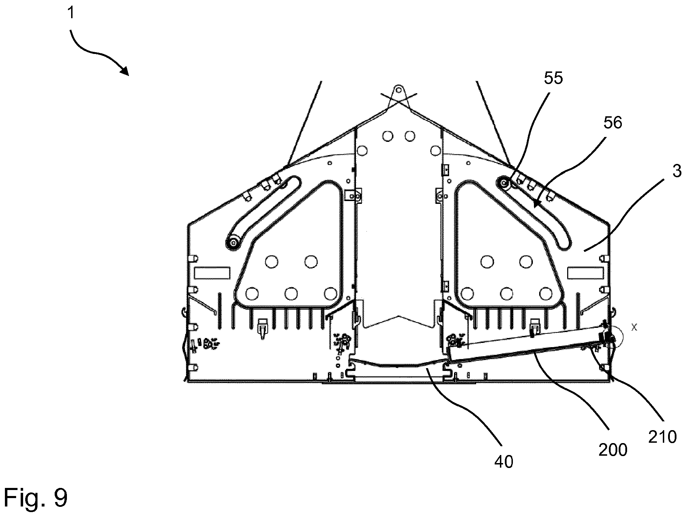

In den Figuren sind gleiche oder im Wesentlichen funktionsgleiche bzw. -ähnliche Elemente mit den gleichen Bezugszeichen bezeichnet. In den Figuren 1 - 12 ist eine beispielhafte Ausführungsform einer erfindungsgemäßen Geflügelaufenthaltsvorrichtung gezeigt. Die Geflügelaufenthaltsvorrichtung 1 erstreckt sich in Längsrichtung von einem ersten Ende 2 zu einem zweiten Ende 4. Entlang der Längsrichtung sind nebeneinander Legenester 30 - 37 angeordnet. Zwei benachbarte Legenester 30 - 37 sind jeweils voneinander durch eine Trennwand 38 getrennt. Die Geflügelaufenthaltsvorrichtung 1 weist im Bereich des ersten Endes 2 eine erste Ecke 10 und eine zweite Ecke 11 auf. An der ersten Ecke 10 ist ein erstes Stützelement 100 angeordnet. Das erste Stützelement 100 erstreckt sich in einer vertikalen Richtung, senkrecht zur Längsrichtung der Geflügelaufenthaltsvorrichtung von einem ersten unteren Standende 101 zu einem ersten oberen Freiende 102.

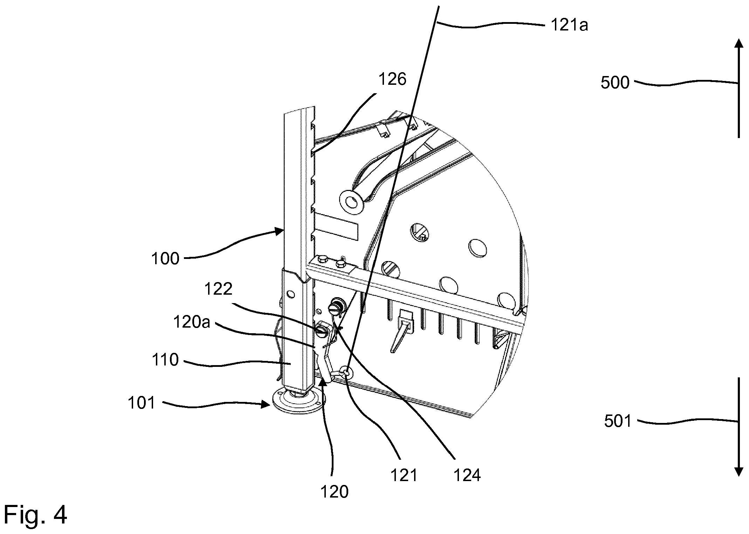

Wie insbesondere in der Figur 4 gezeigt ist, weist das erste Stützelement 100 einen im Wesentlichen viereckigen Querschnitt auf. Eine erste Führungsvorrichtung 110 ist an der ersten Ecke 10 angeordnet. Die erste Führungsvorrichtung 110 bildet eine viereckige Durchgangsöffnung aus, in der das erste Stützelement 100 angeordnet und für eine vertikale Verschiebebewegung relativ zur Führungsvorrichtung geführt ist. Das erste Stützelement 100 weist eine Außenumfangsfläche auf, die zu einer Innenumfangsfläche der Durchgangsöffnung der ersten Führungsvorrichtung 110 korrespondierend ausgebildet ist.

Das erste Stützelement 100 weist eine Vielzahl an Aussparungen 126 auf. Die Aussparungen sind in vertikaler Richtung nebeneinander in einer Reihe angeordnet. Die Geflügelaufenthaltsvorrichtung 1 weist ein als erste Klinke 120 ausgebildetes erstes Verriegelungselement auf, wobei die Klinge 120 derart angeordnet und ausgebildet ist, dass diese mit einem Einrastvorsprung 120a in einer der Aussparungen 126 einrasten kann. Insbesondere sind die Klinke 120 und die Aussparungen 126 derart ausgebildet, dass eine Bewegung des Stützelements 100 in einer nach oben gerichteten vertikalen Standrichtung 500 durch Einrasten des Einrastvorsprungs der Klinke in eine Aussparung gesperrt wird, wohingegen eine Bewegung in einer hierzu entgegengesetzten, nach unten gerichteten vertikalen Freilaufrichtung 501 ermöglicht wird. Zu diesem Zweck ist die Klinke 120 um eine ersten Drehpunkt 122 drehbar angeordnet, der oberhalb des Einrastvorsprungs 120a und seitlich neben dem Stützelement angeordnet ist. Ein Federelement 124 derart angeordnet und ausgebildet, dass dieses eine Federkraft auf die Klinke 120 ausübt, sodass die Klinke stets mit einer Kraft beaufschlagt wird, die den Einrastvorsprung 120a in Richtung des ersten Stützelements 100 drückt. Die Klinke 120 kann hierdurch entgegen der Federkraft der Feder 124 um die Drehachse verschwenken, wenn das Stützelement durch sein Eigengewicht nach unten bewegt wird und hierdurch eine Kraft durch die Ausnehmungen auf den Einrastvorsprung ausübt. Hingegen wird die Klinke 120 in der Ausnehmung selbstverstärkend gehalten, wenn eine nach oben gerichtete Kraft auf das Stützelement wirkt. Das Stützelement 100 kann daher zwar nach unten ausgefahren werden, ist jedoch gegen ein Einfahren nach oben durch die Klinke 120 gesperrt. Das Gewicht des ersten Stützelements 100, die Reibung des ersten Stützelements 100 innerhalb der ersten Führungsvorrichtung 110 und die Federkraft der Feder 124, mit der die Klinke 120 in die Aussparungen 126 gedrückt wird, sind insoweit aufeinander abgestimmt, dass einerseits die Feder 124 die Klinke 120 mit einer ausreichenden Kraft in die Aussparungen 126 drückt und somit ihre Funktion des Einrastens erfüllt und andererseits eine Bewegung des ersten Stützelements 100 in Freilaufrichtung 501 ermöglicht wird, sobald das Stützelement 100 unbelastet ist, indem die Klinke aus der Aussparung herausgedrückt wird. Insbesondere kann die Klinke unter Einwirkung der Gewichtskraft der Geflügelaufenthaltsvorrichtung auf das Stützelement formschlüssig in der Aussparung verriegeln und hierdurch ein unerwünschtes Herausbewegen der Klinke aus der Aussparung verhindern.

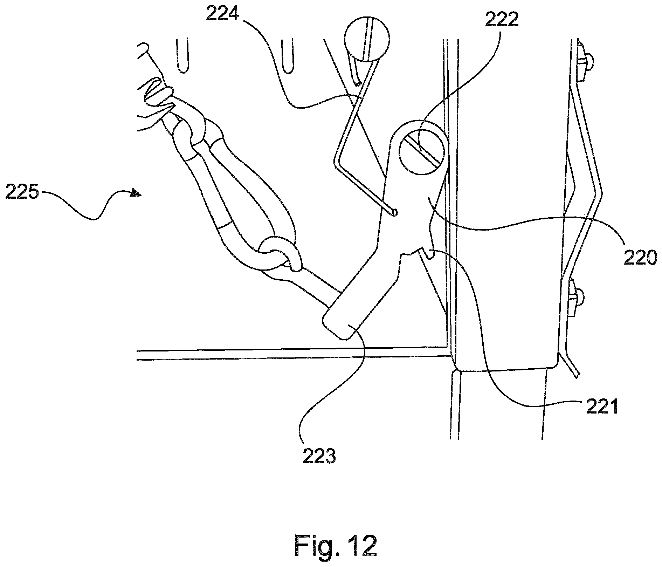

Die Geflügelaufenthaltsvorrichtung 1 weist ferner ein Entsperrelement 121a auf, das an einer an der Klinke 120 befestigten Öse 121 angreift, um die Klinke 120 entgegen der Federkraft zu bewegen. Infolgedessen ist die Klinke 120 nicht in einer der Aussparungen 126 angeordnet und nicht eingerastet. Bei Betätigung des Entsperrelementes 121a ist die Geflügelaufenthaltsvorrichtung 1 in Freilaufrichtung 501 bewegbar und somit absenkbar. Das Ausrasten der Klinke 120 aus einer der Aussparungen 126 kann mittels eines Ständerentlastungselements unterstützt werden, da das Ausrasten der Klinke 120 aus einer der Aussparungen 126 bei unbelastetem Stützelement 100 vereinfacht ist. Das Ständerentlastungselement kann beispielsweise als Seil oder als Bowdenzug ausgebildet sein. Ein weiterer möglicher Entsperrmechanismus 225 ist beispielsweise in der Figur 12 gezeigt.

An der zweiten Ecke 11 ist ein zweites Stützelement 20 angeordnet, das sich von einem zweiten unteren Standende 21 zu einem zweiten oberen Freiende 22 erstreckt und welches analog zum ersten Führungselement 100 in einer zweiten Führungsvorrichtung 23 angeordnet ist, an dessen unteren Standende ein Standelement 24 angeordnet ist und welches mittels eines als zweite Klinke 26 ausgebildeten zweiten Verriegelungselements erfindungsgemäß bewegbar bzw. nicht bewegbar ist.

Die Geflügelaufenthaltsvorrichtung 1 weist ferner ein Dach 50 mit einem First auf, von dem sich Dachelemente aus abschüssig erstrecken. An dem First sind zwei Dachwippen 51 bewegbar angeordnet, die das Aufsitzen eines Geflügeltieres, insbesondere einer Ente, verhindern oder zumindest erschweren. Angrenzend an jede Seite des Firstes ist ein abnehmbares Überdeckungselement 52 angeordnet. Auf der dem First abgewandten Seite des abnehmbaren Überdeckungselements 52 ist ein festes Überdeckungselement 54 angeordnet. Das feste Überdeckungselement 54 ist im Betriebsmodus fest angeordnet und insbesondere nicht dafür vorgesehen, im Betriebsmodus entfernt zu werden.

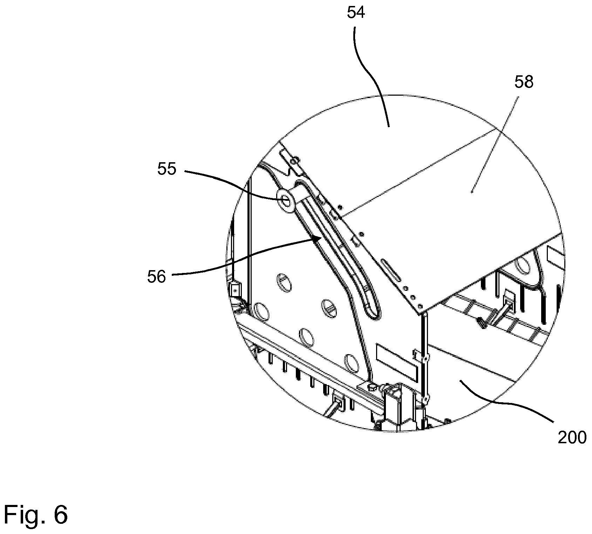

In der Figur 6 ist ferner ein Nestdacherweiterungselement 58 gezeigt, das zusätzlich zu dem abnehmbaren Überdeckungselement 52 und dem festen Überdeckungselement 54 angeordnet ist und das Dach 50 der Geflügelaufenthaltsvorrichtung 1 mit ausbildet. Das Nestdacherweiterungselement 58 kann in Bezug auf eine lichte Höhe über der Bodenfläche variabel montiert werden, so dass dieses ebenfalls zur Einstellung einer Helligkeit im Legenest bzw. eines Schattenwurfs in das Nest genutzt werden kann.

Insbesondere ist es bevorzugt, dass das Nestdacherweiterungselement 58 schwenkbar um eine horizontale Schwenkachse angeordnet ist. Ferner ist es bevorzugt, dass die horizontale Schwenkachse im Wesentlichen parallel zur Längsrichtung der Geflügelaufenthaltsvorrichtung 1 ausgerichtet ist. Ferner vorzugsweise ist die Schwenkachse angrenzend zu einer in Längsrichtung der Geflügelaufenthaltsvorrichtung 1 verlaufenden nach innen weisenden Kante des festen Überdeckungselementes 54 angeordnet.

Das Nestdacherweiterungselement 58 kann auch alternativ oder zusätzlich verschiebbar angeordnet sein. Beispielsweise kann das Nestdacherweiterungselement 58 angeordnet und ausgebildet sein, um unter und/oder über das feste Überdeckungselement 54 und/oder das abnehmbare Überdeckungselement 52 bewegt zu werden.

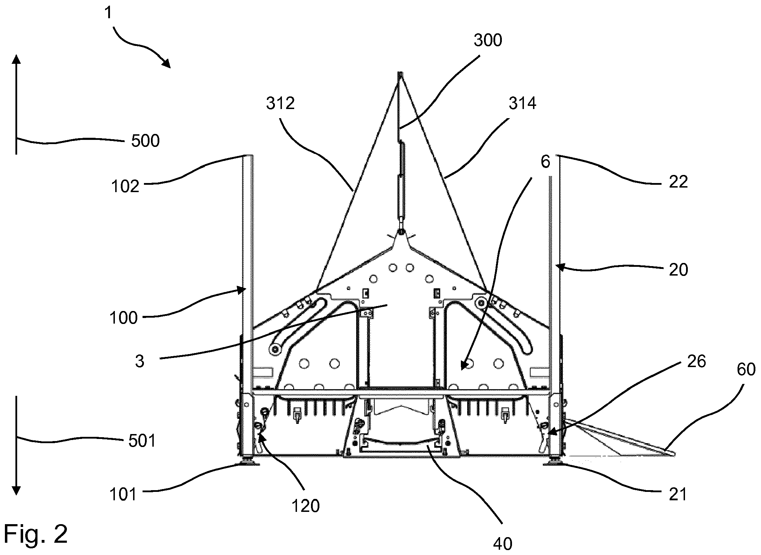

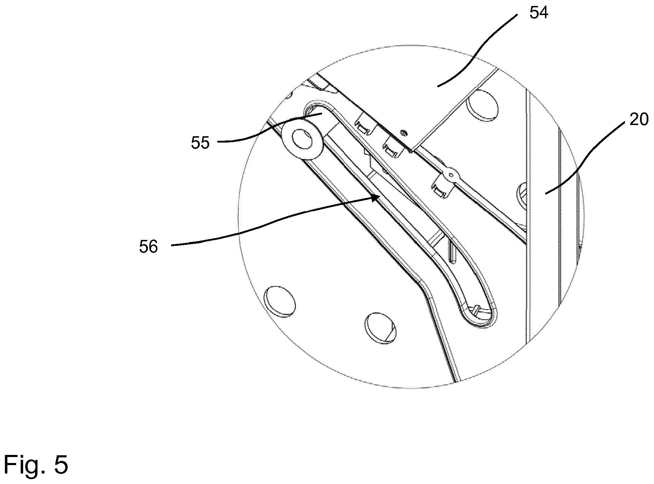

In einer weiteren bevorzugten Ausführungsvariante ist ferner vorgesehen, dass das Nestdacherweiterungselement 58 in einer Richtung vom First zur Traufe eine Tiefe aufweist, und die Tiefe veränderbar ausgebildet ist. Insbesondere ist es bevorzugt, dass das mehrere unterschiedliche Nestdacherweiterungselemente 58 bereitgestellt sind, die verschiedeneTiefen haben und austauschbar sind.In der Figur 5 und der Figur 6 ist gezeigt, wie eine Nestzugangsbarriere realisiert werden kann. Zu diesem Zweck ist an einer Seitenwand am ersten Ende 2 der Geflügelaufenthaltsvorrichtung 1 eine Führungsaussparung 56 angeordnet und vorzugsweise ebenfalls an einer Seitenwand am zweiten Ende 4 der Geflügelaufenthaltsvorrichtung 1 sowie in den Trennwänden 38. Innerhalb der Führungsaussparung 56 kann ein an der als Langrohr ausgebildeten Nestzugangsbarriere angeordneter Führungszapfen 55 geführt werden. Somit ist eine schiebbare Anordnung der Nestzugangsbarriere möglich. In der Figur 1 ist ferner zu erkennen, dass die Nestzugangsbarriere mittels eines Barrierezugelements 310 bewegbar ist, indem dieses durch das feste Überdeckungselement 54 hindurch zur Nestzugangsbarriere reicht. In der Figur 2 ist auf der rechten Seite erkennbar, dass der Führungszapfen 55 in einer oberen Position angeordnet ist. In dieser Position ist die Nestzugangsbarriere in einer oberen Position, so dass ein Zutritt zu dem Nest ermöglicht wird. Auf der linken Seite der Figur 2 ist zu erkennen, dass der Führungszapfen in einer unteren Position angeordnet ist. In dieser Position ist die Nestzugangsbarriere in einer unteren Position, so dass ein Zutritt zu dem Nest im Wesentlichen nicht möglich ist. Um eine Einstellung der Nestzugangsbarrieren zu ermöglichen, umfasst das Barrierezugelement 310 ein erstes Hebeelement 312 und ein zweites Hebeelement 314.

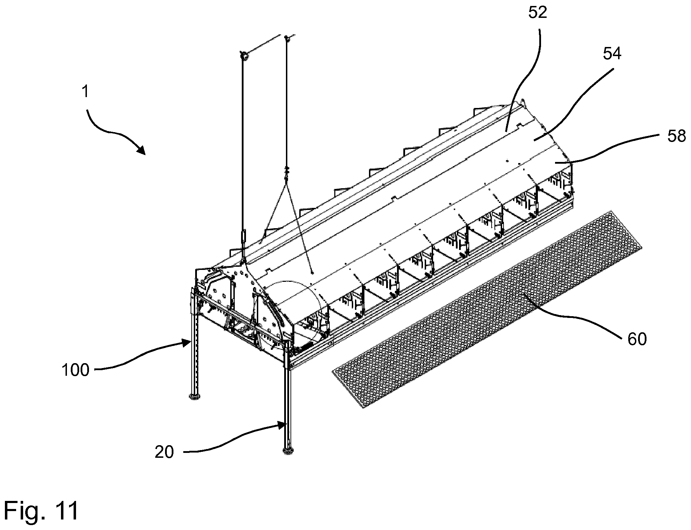

Die Geflügelaufenthaltsvorrichtung ist ferner an einer Nesthebevorrichtung 300 angeordnet, die die Geflügelaufenthaltsvorrichtung 1 anheben kann. In der Figur 11 ist insbesondere die Geflügelaufenthaltsvorrichtung in einem Zustand gezeigt, in dem die Stützelemente 100, 20 ausgefahren sind.

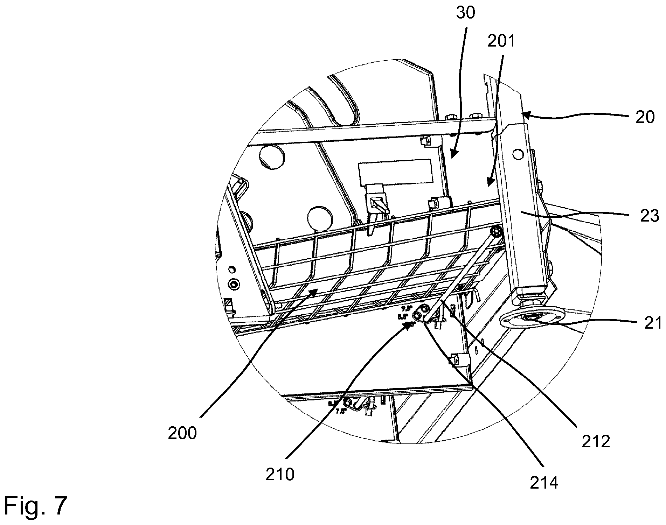

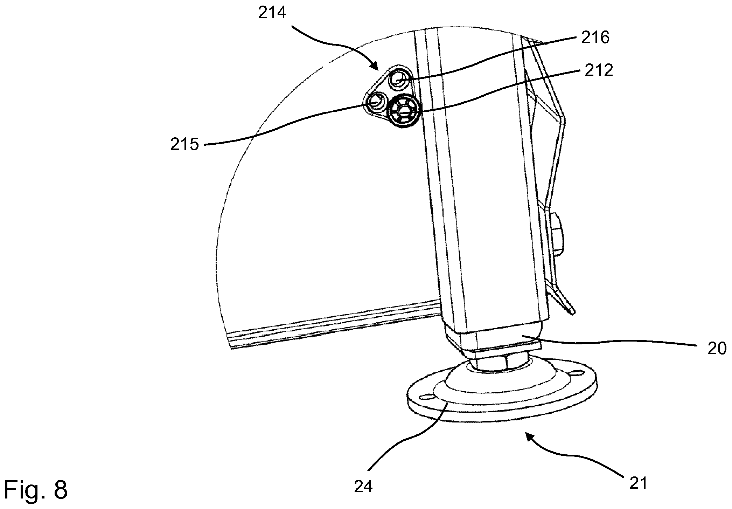

In den Figuren 7 und 8 sind Details der Legenestanordnung gezeigt. Das Legenest 30 umfasst eine kippbare Auftrittsfläche 200. Die kippbare Auftrittsfläche erstreckt sich von einem äußeren Ende, das an einer äußeren Kante der Geflügelaufenthaltsvorrichtung 1 verortet ist, bis zu einem inneren Ende, das hier vorliegend nicht gezeigt ist und sich bis zu einer Eierfördervorrichtung 40 erstreckt. Von dem äußeren Ende 201 zu dem inneren Ende der kippbaren Auftrittsfläche weist dieses vorzugsweise eine Neigung auf. Die Neigung ist vom äußeren zum inneren Ende abschüssig. Durch diese abschüssige Neigung rollt ein innerhalb des Legenests 30 auf der kippbaren Auftrittsfläche 200 abgelegtes Ei in Richtung der Mitte der Geflügelaufenthaltsvorrichtung, wo vorliegend, wie in Figur 1 gezeigt, die Eierfördervorrichtung 40 angeordnet ist.

Der Neigungswinkel der kippbaren Auftrittsfläche 200 ist vorliegend mittels einer Gefälleeinstellvorrichtung 210 veränderbar. Die Gefälleeinstellvorrichtung 210 umfasst eine Einstellstange 212, auf der ein Bereich der kippbaren Auftrittsfläche 200 angrenzend an das äußere Ende 201 aufliegt. Die Einstellstange 212 ist mittels einer Arretiervorrichtung 214 höhenverstellbar. Wie insbesondere in der Figur 8 zu erkennen, umfasst die Arretiervorrichtung 214 ein erstes Arretierloch 215, ein zweites Arretierloch 216 und ein drittes Arretierloch, in dem die Einstellstange 212 angeordnet ist. Durch die Anordnung der Einstellstange 212 in einem höher gelegenen Arretierloch, beispielsweise dem zweiten Arretierloch 216 oder dem ersten Arretierloch 215, wird die Neigung der kippbaren Auftrittsfläche erhöht.

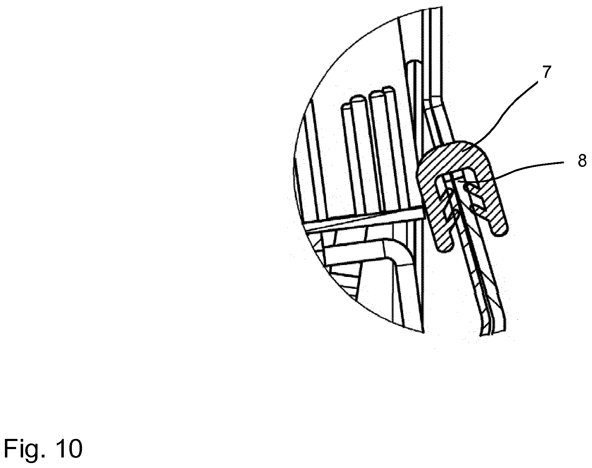

In Figur 10 ist ferner ein Kantenschutzelement 7 gezeigt. Dieses ist insbesondere zum Anordnen an einer Kante vorgesehen, um einen Schutz der Tiere zu ermöglichen. Wenn das Brückenelement beispielsweise aufgrund der Höhe der Geflügelaufenthaltsvorrichtung 1 oder großer Tiere nicht angeordnet werden soll, kann das Kantenschutzelement 7 die Kante derart schützen, dass sich die Tiere an dieser nicht verletzen können.

In der Figur 12 ist die Sperreinrichtung mit einer federnd gelagerten Klinke 220 gezeigt, mit der ein Freilauf realisiert ist. Die Klinke 220 ist um einen Drehpunkt 222 drehbar gelagert. Die Klinke weist ein Einrastende 221 auf, das in nicht gezeigten Aussparungen des Stützelementes anordenbar ist. Das Einrastende 221 ist vom Drehpunkt 222 beabstandet, so dass eine nicht auf den Drehpunkt 222 gerichtete Kraft am Einrastende 221 zu einem Drehmoment um den Drehpunkt 222 führt. Ferner ist ein Federelement 224 angeordnet, das die Klinke 220 beabstandet von dem Drehpunkt 222, insbesondere das Einrastende 221, mit einer Kraft in Richtung der Aussparungen beaufschlagt. An einem Ausrastende 223 ist ferner ein Ausrastzug 225 angeordnet, der angeordnet und ausgebildet ist, die Klinke 220 derart zu bewegen, dass sich insbesondere das Einrastende 221 entgegen der Federkraft bewegt und somit ein Ausrasten der Klinke 220 aus der Aussparung bewirkt wird und das Stützelement in eine Stützrichtung bewegbar ist. Das Ausrastende 223 ist ebenfalls von dem Drehpunkt 222 beabstandet, so dass eine Kraft am Ausrastende 223 ein Moment um den Drehpunkt 222 bewirkt.

Die Geflügelaufenthaltsvorrichtung 1 kann aufgrund des ersten Stützelements 100 und des zweiten Stützelements 20 mit geringem Aufwand in der Höhe verstellt werden. Darüber hinaus kann die Verstellung der Höhe der Geflügelaufenthaltsvorrichtung über einer Aufstellfläche automatisiert und/oder von einer zentralen Kontrollstelle bewirkt werden. Infolgedessen werden die Tiere weniger gestört, finden konstante Nesteintrittsverhältnisse trotz zunehmender Einstreumengen vor und sind somit durch die Geflügelaufenthaltsvorrichtung artgerechter gehalten, sodass das Wohlbefinden der Tiere signifikant gesteigert werden kann.

Neben der Höhenverstellung der Geflügelaufenthaltsvorrichtung kann das Wohlbefinden der Tiere ferner durch die abnehmbaren Überdeckungselemente gesteigert werden. Insbesondere durch die Höhenverstellung der Geflügelaufenthaltsvorrichtung werden die Lichtverhältnisse in den Legenestern gegebenenfalls verändert, da die Lichteinfallswinkel sich durch die Höhenverstellung verändern. Um auch dahingehend gleiche Nestverhältnisse zu schaffen, können durch die abnehmbaren Überdeckungselemente 52 und das verstellbare Nestdacherweiterungselement 58 wiederum gleiche bzw. ähnliche Verhältnisse geschaffen werden. Darüber hinaus können auch die Abrollwinkel von Eiern innerhalb der Legenester verändert werden. Somit kann ergänzend zur verbesserten Produktivität durch die konstanten Nestverhältnisse auch eine verbesserte Eiabführung realisiert werden.

### BEZUGSZEICHEN

- 1: Geflügelaufenthaltsvorrichtung
- 2: erstes Ende
- 3: Seitenwand
- 4: zweites Ende
- 6: Bodenfläche
- 7: Kantenschutzelement
- 8: Seitenblech
- 10: erste Ecke
- 11: zweite Ecke
- 20: zweites Stützelement
- 21: zweites unteres Standende
- 22: zweites oberes Freiende
- 23: zweite Führungsvorrichtung
- 24: Standelement
- 26: zweite Klinke
- 30: erstes Legenest
- 31: zweites Legenest
- 32: drittes Legenest
- 33: viertes Legenest
- 34: fünftes Legenest
- 35: sechstes Legenest
- 36: siebtes Legenest
- 37: achtes Legenest
- 38: Trennwand
- 40: Eierfördervorrichtung
- 50: Dach
- 51: Dachwippe

- 52: abnehmbares Überdeckungselement
- 54: festes Überdeckungselement
- 55: Führungszapfen
- 56: Führungsaussparung
- 58: Nestdacherweiterungselement
- 60: Brückenelement
- 100: erstes Stützelement
- 101: erstes unteres Standende
- 102: erstes oberes Freiende
- 110: erste Führungsvorrichtung
- 120: erste Klinke
- 120a: Einrastvorsprung
- 121: Öse
- 122: erster Drehpunkt
- 124: Federelement
- 126: Aussparung
- 200: kippbare Auftrittsfläche
- 201: äußeres Ende
- 210: Gefälleeinstellvorrichtung
- 212: Einstellstange
- 214: Arretiervorrichtung
- 215: erstes Arretierloch
- 216: zweites Arretierloch
- 121: Einrastende
- 121a: Entsperrelement
- 123: Ausrasthebel
- 220: Klinke
- 221: Einrastende

- 222: Drehpunkt
- 223: Ausrastende
- 224: Federelement
- 225: Ausrastzug
- 300: Nesthebevorrichtung
- 310: Barrierezugelement
- 312: erstes Hebeelement
- 314: zweites Hebeelement
- 500: Standrichtung
- 501: Freilaufrichtung

## Patentansprüche

1. Geflügelaufenthaltsvorrichtung (1), insbesondere zur Eiablage für Enten, umfassend:
- eine Bodenfläche (6),
- eine Mehrzahl von nebeneinander angeordneten Legenestern (30, 31, 32), die oberhalb der Bodenfläche angeordnet sind,
- mindestens ein Stützelement (100; 20), das sich in vertikaler Richtung von einem unteren Standende (101; 21) zu einem oberen Freiende (102; 22) erstreckt und in vertikaler Richtung relativ bewegbar zu der Bodenfläche angeordnet ist, und
- ein Verriegelungselement (120, 120a, 122, 124, 126), welches mit dem Stützelement zusammenwirkt, um eine Bewegung des Stützelements in einer vertikal nach oben gerichteten Standrichtung (500) relativ zu der Bodenfläche zu sperren und eine Bewegung des Stützelements relativ zu der Bodenfläche in einer vertikal nach unten gerichteten Freilaufrichtung (501) freizugeben, umfassend einen Freilauf, der das Einfahren des Stützelements in Standrichtung sperrt und das Ausfahren des Stützelements in Freilaufrichtung ermöglicht, um die Höhe der Bodenfläche und der Legenester oberhalb einer Aufstellfläche, auf der die Geflügelaufenthaltsvorrichtung aufgestellt ist, anzupassen.

2. Geflügelaufenthaltsvorrichtung nach dem vorhergehenden Anspruch,
- wobei die Bodenfläche durch eine Gitter- und/oder Streben- und/oder Gestellanordnung ausgebildet ist und eine drei-, vier- oder mehreckige Geometrie aufweist,
- wobei vorzugsweise in einem Bereich angrenzend
o an eine erste Ecke ein erstes Stützelement, und/oder
o an eine zweite Ecke ein zweites Stützelement, und/oder
o an eine dritte Ecke ein drittes Stützelement, und/oder
o gegebenenfalls an eine vierte Ecke ein viertes Stützelement angeordnet ist.

3. Geflügelaufenthaltsvorrichtung nach einem der vorhergehenden Ansprüche, umfassend ein Entsperrelement (121a), das angeordnet und ausgebildet ist, eine Bewegung des mindestens einen Stützelementes in Standrichtung zu ermöglichen.

4. Geflügelaufenthaltsvorrichtung nach einem der vorhergehenden Ansprüche, umfassend
- eine Führungsvorrichtung (110) für das mindestens eine Stützelement,
- wobei die Führungsvorrichtung insbesondere eine rohrförmige Geometrie mit einem Innenquerschnitt aufweist,
- das mindestens eine Stützelement einen Außenquerschnitt aufweist, wobei der Innenquerschnitt der Führungsvorrichtung und der Außenquerschnitt des Stützelements insbesondere die gleiche Geometrie aufweisen, und
- der Innenquerschnitt der Führungsvorrichtung und der Außenquerschnitt des Stützelements insbesondere derart ausgebildet sind, dass das mindestens eine Stützelement bewegbar innerhalb der Führungsvorrichtung anordenbar ist.

5. Geflügelaufenthaltsvorrichtung nach einem der vorhergehenden Ansprüche, wobei
- in einem Standmodus
o das untere Standende des mindestens einen Stützelements auf einer Aufstellfläche angeordnet ist,
o das Gewicht der Geflügelaufenthaltsvorrichtung zumindest teilweise auf dem mindestens einen Stützelement lastet, und
o das mindestens eine Stützelement die Geflügelaufenthaltsvorrichtung mit einem definierten Abstand über der Aufstellfläche abstützt, und
- in einem Einstellmodus
o das Gewicht der Geflügelaufenthaltsvorrichtung im Wesentlichen nicht auf dem mindestens einen Stützelement lastet, und
o das mindestens eine Stützelement in Freilaufrichtung im Wesentlichen durch Eigengewicht bewegbar angeordnet ist.

6. Geflügelaufenthaltsvorrichtung nach einem der vorhergehenden Ansprüche, umfassend ein Brückenelement (60), das angeordnet und ausgebildet ist, einem Geflügeltier den Zugang zu der Bodenfläche zu ermöglichen, wobei das Brückenelement vorzugsweise eine flächige und/oder gestufte Oberfläche aufweist und sich ferner vorzugsweise von einer Kante der Geflügelaufenthaltsvorrichtung ausgehend nach schräg unten erstreckt.

7. Geflügelaufenthaltsvorrichtung nach einem der vorhergehenden Ansprüche, umfassend ein Überdeckungselement, wobei
- das Überdeckungselement (52; 54) im Betriebsmodus zumindest abschnittsweise in vertikaler Richtung über der Bodenfläche angeordnet ist.

8. Geflügelaufenthaltsvorrichtung nach einem der vorhergehenden Ansprüche, wobei
- an einer Seite des mindestens einen Legenests eine Eiersammelvorrichtung (40) angeordnet ist,
- wobei Legeabschnitte der Legenester in vertikaler Richtung von der Eiersammelvorrichtung beabstandet sind, wobei ein Gefälle von dem Legeabschnitt zu der Eiersammelvorrichtung einstellbar ausgebildet ist.

9. Geflügelaufenthaltsvorrichtung nach einem der vorhergehenden Ansprüche, wobei die Legenester jeweils zumindest abschnittsweise als ein um mindestens eine horizontale Achse kippbares Element ausgebildet sind.

10. Geflügelaufenthaltsvorrichtung nach einem der vorhergehenden Ansprüche, umfassend eine Nestzugangsbarriere, die angeordnet und ausgebildet ist, um den Zugang zu mindestens einem der Legenester für die Geflügeltiere zu versperren.

11. Geflügelaufenthaltsvorrichtung nach einem der vorhergehenden Ansprüche, umfassend eine sich entlang einer Seite im Bereich der Bodenfläche erstreckende Seitenschiene, die eine oder mehrere Aussparungen im Bereich der Legenester aufweist

12. Geflügelstall, insbesondere zur Produktion von Enteneiern, umfassend eine Geflügelaufenthaltsvorrichtung nach mindestens einem der Ansprüche 1-9, wobei
- die Geflügelaufenthaltsvorrichtung an einer Nesthebevorrichtung (312, 314) angeordnet ist, die angeordnet und ausgebildet ist, die Geflügelaufenthaltsvorrichtung relativ zu einer Aufstellfläche derart zu bewegen, dass ein Abstand zu dieser Aufstellfläche vergrößert wird.

13. Verfahren zur Haltung von Enten, umfassend
- Bereitstellen einer Geflügelaufenthaltsvorrichtung nach einem der Ansprüche 1-11, und/oder eines Geflügelstalls nach Anspruch 12,
- Bewegen der Geflügelaufenthaltsvorrichtung in einem Einstellmodus in einer im Wesentlichen vertikalen Richtung, wobei ein Abstand zwischen einer Aufstellfläche und der Bodenfläche vergrößert wird,
- wobei das mindestens eine Stützelement durch sein Eigengewicht im Einstellmodus ausfährt und die Geflügelaufenthaltsvorrichtung in einem anschließenden Standmodus mit einem definierten Abstand zwischen der Aufstellfläche und der Bodenfläche abstützt.

## Claims

1. A poultry containment (1), in particular for laying eggs for ducks, comprising:
- a bottom surface (6),
- a plurality of side-by-side laying nests (30, 31, 32) arranged above the bottom surface,
- at least one support member (100; 20) extending vertically from a lower standing end (101; 21) to an upper free end (102; 22) and being vertically movable relative to the floor surface, and
- a locking member (120, 120a, 122, 124, 126) cooperating with the support member to lock movement of the support member in a vertically upward standing direction (500) relative to the floor surface and to allow movement of the support member relative to the floor surface in a vertically downwardly directed freewheeling direction (501), comprising a one-way clutch that prevents the supprt member from retracting in the standing direction and allows the extension of the support member in the freewheeling direction in order to reduce the height of the bottom surface and the laying nests above a support surface on which the poultry containment is put up.

2. The poultry containment according to the preceding claim,
- wherein the bottom surface is formed by a grid and/or strut and/or frame arrangement and has a triangular, quadrilateral or polygonal geometry,
- whereby preferably in an area adjacent
- to a first corner a first support member, and/or
- to a second corner a second support member, and/or
- to a third corner a third support member, and/or
- If necessary, to a fourtrh corner a fourth support member is arranged.

3. The poultry containment according to any one of the preceding claims, comprising an unlocking member (121a) arranged and adapted to allow movement of the at least one support member in the standing direction.

4. The poultry containment according to any one of the preceding claims, comprising
- a guiding device (110) for the at least one support member,
- wherein the guiding device has, in particular, a tubular geometry with an internal cross section,
- the at least one support member has an outer cross-section, wherein the inner cross-section of the guiding device and the outer cross-section of the support member in particular have the same geometry, and
- the internal cross-section of the guiding device and the external cross-section of the support member are designed in particular in such a way that the at least one support member can be arranged movably within the guiding device.

5. The poultry containment according to any one of the preceding claims-
- wherein a stand mode
- the lower standing end of the at least one support member is arranged on a support surface,
- the weight of the poultry containment rests at least partially on the at least one support member, and
- at least one support member supports the poultry containment in a defined distance above the installation surface, and
- in a setting mode
- the weight of the poultry containment does not substantially rest on the at least one support member, and
- the at least one support member is arranged to be movable in the freewheeling direction essentially by its own weight.

6. The poultry containment according to any one of the preceding claims, comprising a bridge member (60) arranged and configured to allow a poultry animal access to the support surface, said bridge member preferably having a flat and/or stepped surface, and further preferably extending downward from one edge of the vessel containment device.

7. The poultry containment according to any one of the preceding claims, comprising a top cover element, wherein
- the top cover element (52; 54) is arranged in the operating mode at least in sections in the vertical direction above the bottom surface.

8. The poultry containment according to any of the preceding claims,
wherein
- on one side of the at least one laying nest an egg collecting device (40) is arranged,
- wherein laying portions of the laying nests are spaced apart from the egg collecting device in the vertical direction, wherein a slope from the laying portion to the egg collecting device is adjustably formed.

9. The poultry containment according to one of the preceding claims, wherein the laying nests are each formed at least in sections as an element which can be tilted about at least one horizontal axis.

10. The poultry containment according to any one of the preceding claims, comprising a nest access barrier arranged and configured to block access to at least one of the laying nests tor the poultry animals.

11. The poultry containment according to any one of the preceding claims, comprising a side wall extending along one side in the region of the bottom surface, said side wall having opnings in the region of the laying nests.

12. A poultry house, in particular for the production of duck eggs, comprising a poultry containment according to at least one of claims 1-9, wherein
- the poultry containment is arranged on a nest lifting device (312, 314) which is arranged and constructed to move the poultry containment relative to a support surface in such a way that a distance to this support surface is increased.

13. Method of keeping ducks, comprising.
- Providing a poultry containment according to any one of claims 1-11, and/or a poultry house according to claim 12,
- Moving the poultry containment in a setting mode in a substantially vertical direction, wherein a distance between a support surface and the support surface is increased,
- wherein the at least one support member moves out by its own weight in the setting mode and supports the poultry containment in a subsequent standing mode in a defined distance between the support surface and the bottom surface.

## Revendications

1. Dispositif d'abritement de volailles (1), en particulier destiné au dépôt d'œufs pour canards, comprenant :
- une face au sol (6),
- une multitude de nids (30, 31, 32) disposés côte à côte, qui sont disposés au-dessus de la face au sol,
- au moins un élément de soutien (100 ; 20), qui s'étend dans une direction verticale depuis une extrémité d'appui inférieure (101 ; 21) vers une extrémité libre supérieure (102 ; 22) et est disposé dans une direction verticale de manière à pouvoir être déplacé vers la face au sol, et
- un élément de verrouillage (120, 120a, 122, 124, 126), lequel coopère avec l'élément de soutien pour bloquer un déplacement de l'élément de soutien dans une direction d'appui (500) dirigée verticalement vers le haut par rapport à la face au sol et pour libérer un déplacement de l'élément de soutien par rapport à la face au sol dans une direction de roue libre (501) dirigée verticalement vers le bas, comprenant une roue libre, qui bloque l'entrée de l'élément de soutien dans la direction d'appui et permet la sortie de l'élément de soutien dans la direction de roue libre pour adapter la hauteur de la face au sol et des nids au-dessus d'une face de placement, sur laquelle le dispositif d'abritement de volailles est placé.

2. Dispositif d'abritement de volailles selon la revendication précédente,
- dans lequel la face au sol est réalisée par un ensemble grillagé et/ou un ensemble d'entretoises et/ou un ensemble d'armatures et présente une géométrie triangulaire, rectangulaire ou polygonale,
- dans lequel est disposé de préférence dans une zone jouxtant
- - un premier coin, un premier élément de soutien et/ou
- - un deuxième coin, un deuxième élément de soutien, et/ou
- - un troisième coin, un troisième élément de soutien, et/ou
- - éventuellement un quatrième coin, un quatrième élément de soutien.

3. Dispositif d'abritement de volailles selon l'une quelconque des revendications précédentes, comprenant un élément de déblocage (121a) qui est disposé et réalisé pour permettre un déplacement de l'au moins un élément de soutien dans la direction d'appui.

4. Dispositif d'abritement de volailles selon l'une quelconque des revendications précédentes, comprenant
- un dispositif de guidage (110) pour l'au moins un élément de soutien,
- dans lequel le dispositif de guidage présente en particulier une géométrie tubulaire avec une section transversale intérieure,
- l'au moins un élément de soutien présente une section transversale extérieure, dans lequel la section transversale intérieure du dispositif de guidage et la section transversale extérieure de l'élément de soutien présentent en particulier la même géométrie, et
- la section transversale intérieure du dispositif de guidage et la section transversale extérieure de l'élément de soutien sont réalisées en particulier de telle manière que l'au moins un élément de soutien peut être disposé de manière à pouvoir être déplacé à l'intérieur du dispositif de guidage.

5. Dispositif d'abritement de volailles selon l'une quelconque des revendications précédentes, dans lequel
- dans un mode d'appui
- - l'extrémité d'appui inférieure de l'au moins un élément de soutien est disposée sur une face de placement,
- - le poids du dispositif d'abritement de volailles exerce au moins partiellement une charge sur l'au moins un élément de soutien,
- - l'au moins un élément de soutien supporte le dispositif d'abritement de volailles avec une distance définie au-dessus de la face de placement, et
- dans un mode de réglage
- - le poids du dispositif d'abritement de volailles n'exerce pas sensiblement une charge sur l'au moins un élément de soutien, et
- - l'au moins un élément de soutien est disposé de manière à pouvoir être déplacé dans la direction de roue libre sensiblement par son propre poids.

6. Dispositif d'abritement de volailles selon l'une quelconque des revendications précédentes, comprenant un élément formant pont (60) qui est disposé et réalisé pour permettre à une volaille l'accès à la face au sol, dans lequel l'élément formant pont présente de préférence une surface plate et/ou étagée et s'étend en outre de préférence en partant d'une arête du dispositif d'abritement de volailles vers le bas à l'oblique.

7. Dispositif d'abritement de volailles selon l'une quelconque des revendications précédentes, comprenant un élément de recouvrement, dans lequel
- l'élément de recouvrement (52 ; 54) est disposé dans le mode de fonctionnement au moins par endroits dans une direction verticale au-dessus de la face au sol.

8. Dispositif d'abritement de volailles selon l'une quelconque des revendications précédentes, dans lequel
- un dispositif de collecte d'œufs (40) est disposé sur un côté de l'au moins un nid,
- dans lequel des sections de pose des nids sont tenues à distance dans la direction verticale du dispositif de collecte d'œufs, dans lequel une déclivité depuis la section de pose vers le dispositif de collecte d'œufs est réalisée de manière à pouvoir être réglée.

9. Dispositif d'abritement de volailles selon l'une quelconque des revendications précédentes, dans lequel les nids sont réalisés respectivement au moins par endroits en tant qu'un élément pouvant être basculé autour d'au moins un axe horizontal.

10. Dispositif d'abritement de volailles selon l'une quelconque de revendications précédentes, comprenant une barrière d'accès au nid qui est disposée et réalisée pour obstruer l'accès à au moins un des nids pour les volailles.

11. Dispositif d'abritement de volailles selon l'une quelconque des revendications précédentes, comprenant un rail latéral s'étendant le long d'un côté dans la zone de la face au sol, qui présente un ou plusieurs évidements dans la zone des nids.

12. Poulailler, en particulier pour la production d'œufs de canard, comprenant un dispositif d'abritement de volailles selon au moins l'une quelconque des revendications 1 - 9, dans lequel
- le dispositif d'abritement de volailles est disposé sur un dispositif de levage de nid (312, 314) qui est disposé et réalisé pour déplacer le dispositif d'abritement de volailles par rapport à une face de placement de telle manière qu'une distance par rapport à ladite face de placement est agrandie.

13. Procédé pour l'élevage de canards, comprenant
- la fourniture d'un dispositif d'abritement de volailles selon l'une quelconque des revendications 1 - 11 et/ou d'un poulailler selon la revendication 12,
- le déplacement du dispositif d'abritement de volailles dans un mode de réglage dans une direction sensiblement verticale, dans lequel une distance entre une face de placement et la face au sol est agrandie,
- dans lequel l'au moins un élément de soutien sort du fait de son propre poids dans le mode de réglage et soutient le dispositif d'abritement de volailles, dans un mode d'appui qui suit à une distance définie entre la face de placement et la face au sol.
